# EUROPEAN PATENT APPLICATION

(11) **EP 2 778 168 A1**
(43) Date of publication of application: **17.09.2014**
(21) Application number: 12840822.6
(22) Date of filing: 10.10.2012
(51) Int. Cl.: C07D 487/04, H01L 51/30, H01L 51/05, H01L 51/00

(54) **PYRROLO PYRROLEDIONE-THENEQUINONE COMPOUND, AND PREPARATION PROCESS AND USE THEREOF**

(30) Priority: 11.10.2011 CN 201110306463
(71) Applicant: Institute Of Chemistry, Chinese Academy Of Sciences, Beijing 100190 (CN)
(72) Inventor: QIAO, Yali, Beijing 100190 (CN); ZHANG, Jing, Beijing 100190 (CN); XU, Wei, Beijing 100190 (CN); ZHU, Daoben, Beijing 100190 (CN)
(74) Representative: Langfinger, Klaus Dieter
(86) International application number: PCT/CN2012/001366
(87) International publication number: WO 2013/053202

(57) **Abstract**

Disclosed are a pyrrolo-pyrroledione-thiophenequinone compounds as shown in formula I, a preparation process thereof and the use thereof as an organic semiconductor material. The preparation process for the compound of formula I comprises reacting NaH, an α-bromine substituted pyrrolo-pyrroledione-thiophene oligomer as shown by formula II and malononitrile sodium salt, in the presence of catalytic Pd(PPh₃)₄, and then adding to the reaction system saturated bromine water to carry out an oxidation reaction, so as to obtain the compound of formula I. The compound of formula I has a good field effect performance, an electron mobility of above 0.1 cm²V⁻¹s⁻¹, an on/off current ratio up to 10⁵, and is stable in air.

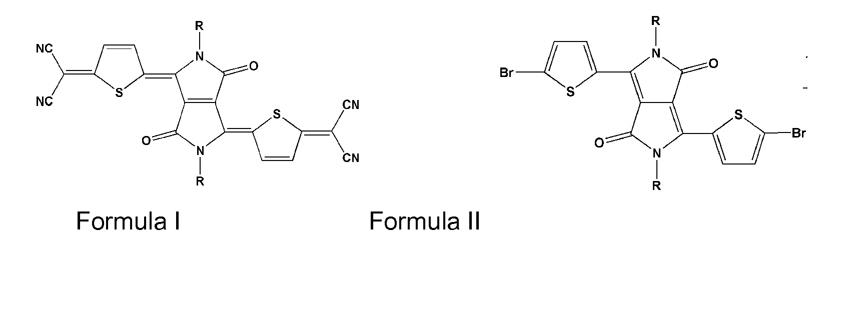

## Description

### Technical Field

The present invention relates to organic semiconductor materials for field effect transistors, particularly to pyrrolo-pyrroledione- thiophenequinone compounds, a process for their manufacture and the use thereof.

### Technical Background

Organic Field Effect Transistor (OFET) has caused wide concern after it first appeared in 1986 (Tsumura, A.; Koezuka, H.; Ando, T. Appl. Phys. Lett. 1986, 49, 1210) because of its potential application values in large area sensor, RFID tag, electronic paper and large screen display. Compared with inorganic transistors, OFETs have the advantages of low-cost, light-weight and good flexibility. In recent years, OFETs have developed quickly and became one of the most important organic electronic devices (Zaumseil, J., Sirringhaus, H., Chem. Rev. 2007, 107, 1296-1323; Murphy, A. R., Frechet, J. M. J., Chem. Rev. 2007, 107, 1066-1096; Sun Y., Liu Y. Q., Zhu D. B., J. Mater. Chem., 2005, 15, 53-65.).

Currently, p-type organic semiconductors have not only been comparable with the traditional inorganic Si-based material in the aspect of field effect mobility (Klauk, H., Halik, M., Zschieschang, U., Schmid, G., Radlik, W., Weber, W., J. Appl. Phys. 2002, 92, 5259-5263 ; Katz, H. E., Chem. Mater. 2004, 16, 4748-4756.), but also achieved a good stability in air. However, the n-type organic semiconductors usually have a relatively low field effect mobility and a bad air stability. Thus their development is behind p-type semiconductors. Recently, since n-type semiconductors play an important role in constructing bipolar transistors and logic complementary circuits, high-performance n-type semiconductor materials stable in air have aroused more and more interest of the researchers (Usta, H., Risko, C., Wang, Z. M., Huang, H., Deliomeroglu, M. K., Zhukhovitskiy, A., Facchetti, A., Marks, T. J., J. Am. Chem. Soc. 2009, 131, 5586-5608; Ling, M. M., Erk, P., Gomez, M., Koenemann, M., Locklin, J., Bao, Z. N., Adv. Mater. 2007, 19, 1123-1127; Gsanger, M., Oh, J. H.; Konemann, M., Hoffken, H. W., Krause, A. M., Bao, Z. N., Wurthner, F., Angew. Chem. Int. Edit. 2010, 49, 740-743.). The number of reports about solution processible n-type semiconductor materials with high performance and air stability increases rapidly (Gao, X. K., Di, C. A., Hu, Y. B., Yang, X. D., Fan, H. Y., Zhang, F., Liu, Y. Q., Li, H. X., Zhu, D. B., J. Am. Chem. Soc. 2010, 132, 3697-3699; Hu, Y. B., Gao, X. K., Di, C. A., Yang, X. D., Zhang, F., Liu, Y. Q., Li, H. X., Zhu, D. B., Chem. Mater. 2011, 23, 1204-1215.), and the design and synthesis of n-type organic semiconductors has become a hot research area.

TCNQ-type compounds which are based on quinone-type structure of thiophene oligomers and which are used as novel n-type semiconductor materials have excellent overall performance: high performance, stable in air, and some of them are solution processible. Therefore, such TCNQ-type compounds show good application prospects in OFETs (Handa, S., Miyazaki, E., Takimiya, K., Kunugi, Y., J. Am. Chem. Soc. 2007, 129, 11684-11685; Suzuki, Y., Miyazaki, E., Takimiya, K., J. Am. Chem. Soc. 2010, 132, 10453-10466; Suzuki, Y., Shimawaki, M., Miyazaki, E., Osaka, I., Takimiya, K., Chem. Mater. 2011, 23, 795-804.). Nowadays, the design of this kind of materials mainly includes: modifying the mother nucleus of oligomeric thiophenes with a soluble group or adjusting the types of substituents on the ends to improve the solubility of the materials and the orderliness of the accumulation of molecules in thin film so as to achieve the final purpose of increasing the overall performance of the materials. However, there are no reports on optimizing molecule performance through changing the structure of the mother nucleus of oligomeric thiophenes.

### Contents of Invention

An object of present invention is to provide pyrrolo-pyrroledione-thiophenequinone compounds, a preparation process and the use thereof.

The general formula of pyrrolo-pyrroledione-thiophenequinone compounds provided in the present application is shown by Formula I:

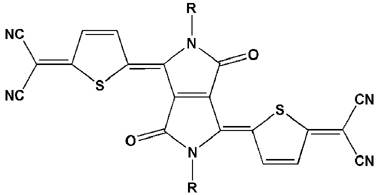

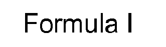

In Formula I, R is hydrogen, alkyl with a total number of 8 to 20 carbon atoms or alkoxy with a total number of 8 to 20 carbon atoms, preferably branched alkyl with a total number of 8 carbon atoms or branched alkyl with a total number of 16 carbon atoms.

The process of preparing the compounds represented by Formula I provided in the present invention comprises the following steps: under the catalytic action of Palladium(O) tetrakis(triphenylphosphine) (Pd(PPh₃)₄), mixing sodium hydride, α-bromine substituted pyrrolo-pyrroledione-thiophene oligomer represented by Formula II and malononitrile sodium salt uniformly to carry out nucleophilic substitution reaction to form a divalent anionic intermediate, and then adding saturated bromine water into the reaction system to carry out an oxidation reaction. The compound represented by Formula I is obtained after the reactions.

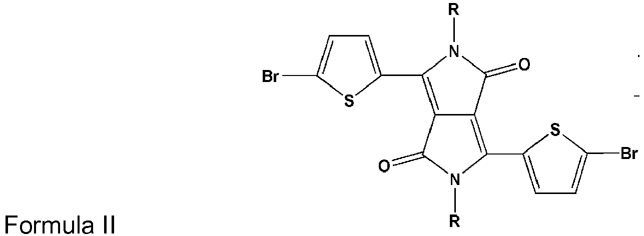

In Formula II, R is hydrogen, alkyl with a total number of 8 to 20 carbon atoms or alkoxy with a total number of 8 to 20 carbon atoms, preferably R is branched alkyl with a total number of 8 carbon atoms or branched alkyl with a total number of 16 carbon atoms.

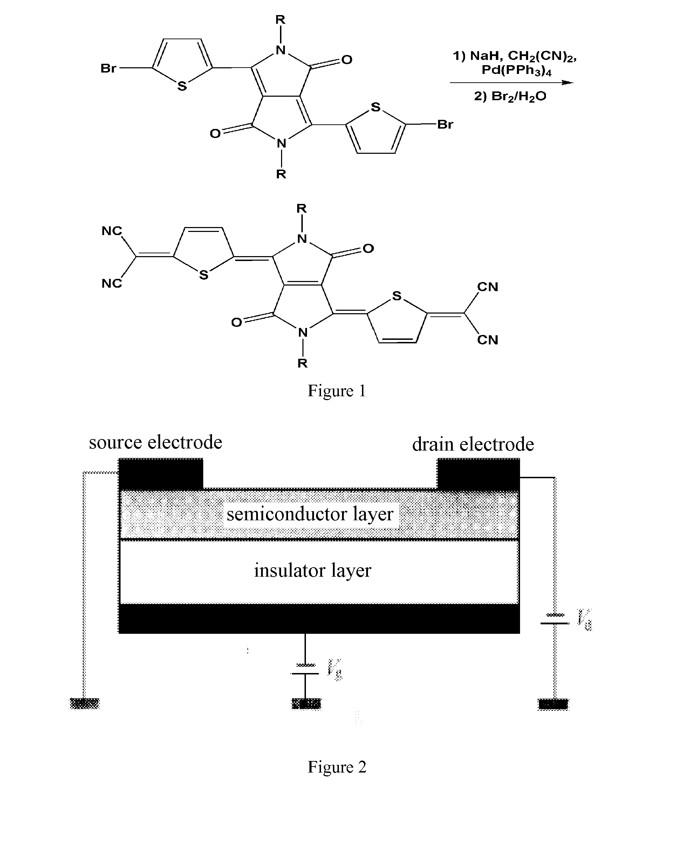

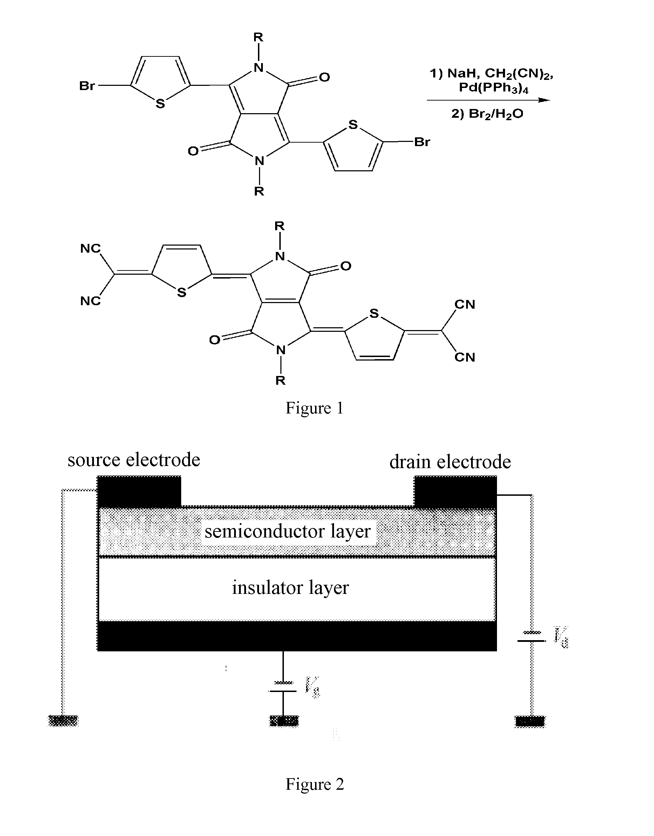

The reaction scheme of this process is shown in Figure 1.

In the process, the ratio of sodium hydride, α-bromine substituted pyrrolo-pyrroledione-thiophene oligomer represented by Formula II, malononitrile sodium salt and saturated bromine water is 4.63-5.56 mmol: 0.58-0.61 mmol: 1.39-1.45 mmol: 25-30 mL, preferably 4.63 mmol: 0.58 mmol: 1.39 mmol: 25 mL.

In the step of nucleophilic substitution reaction (namely, Takahashi coupling reaction), the temperature is 90-110°C, preferably 100°C. The time is 4-6 hours, preferably 4.5 hours. This nucleophilic substitution reaction step achieves the substitution for α-position of thiophene in precursory compound α-bromine substituted pyrrolo-pyrroledione-thiophene oligomer by malononitrile group.

In the step of oxidation reaction, the temperature is 0-25°C, preferably 25°C. The time is 2-3 hours, preferably 2.5 hours.

Both the nucleophilic substitution reaction and the oxidation reaction are carried out in a solvent and under an inert atmosphere. Said solvent is selected from at least one of ethylene glycol dimethyl ether, tetrahydrofuran and N,N-dimethylformamide dried by sodium, preferably ethylene glycol dimethyl ether. Said inert atmosphere is nitrogen atmosphere or argon atmosphere.

The reactant malononitrile sodium salt used in this process is formed in situ from sodium hydride and malononitrile through a conventional method. The separation of a dark purple precipitate from the reaction system indicates the formation of the divalent anionic intermediate.

The process of preparing the compounds represented by Formula I provided above can further comprise the following steps: when the oxidation reaction is finished, add dichloromethane into the reaction system for extraction; combine organic phases after extraction; wash the organic phase with saturated sodium chloride aqueous solution, dry the organic phase and carry out column chromatography. The eluent used is the mixed solution of petroleum ether and dichloromethane with the volume ratio of 1:2. Recrystallization with dichloromethane after the column chromatography can be conducted to obtain the purified compounds represented by Formula I.

The semiconductor layer of an organic field effect transistor formed from the compound represented by Formula I provided in the present invention is also within the scope of the present invention.

The present invention also provides a n-type organic field effect transistor which is composed of, from bottom to up, substrate, insulator layer, semiconductor layer, and source electrode layer and drain electrode layer located in the same layer; said source electrode layer and drain electrode layer are not in contact with each other; wherein the material forming said semiconductor layer is a compound of Formula I provided in the present invention.

The material forming the substrate of the organic field effect transistor is selected from at least one of glass, ceramic and silicon wafer, preferably silicon wafer.

The material forming said insulator layer is selected from at least one of silica, n-octadecyltrichlorosilane modified silica, aluminium oxide, polyvinylpyrrolidone and polymethylmethacrylate, preferably silica. The thickness of said insulator layer is 300-500 nm, preferably 500 nm. As for n-octadecyl trichlorosilane (OTS) modified silica, the modification method of OTS is a conventional method and is OTS monomolecular layer modification.

The material forming said source electrode layer and drain electrode layer is selected from at least one of gold, silver and aluminum, preferably gold. The thickness of source electrode layer or drain electrode layer is 20-30 nm, preferably 30 nm.

The thickness of said semiconductor layer is 50-80 nm, preferably 50 nm.

The aforesaid n-type organic field effect transistor can be produced by a conventional preparation method. For example, the method can include the following steps: preparing on the substrate the following layers from bottom to up: the insulator layer, the semiconductor layer, and the source electrode layer and drain electrode layer so as to provide said n-type organic field effect transistor.

In the method, in the step of preparing said insulator layer, the preparation includes in situ thermal growth or plasma enhanced chemical vapor deposition. In the step of preparing said semiconductor layer, the preparation includes spin coating, drop-casting or vacuum evaporation. In the step of preparing said source electrode layer and drain electrode layer, the preparation includes vacuum evaporation, plasma enhanced chemical vapor deposition or printing.

The present invention provides quinone-type compounds with pyrrolo-pyrroledione-thiophene oligomers as mother nucleus and terminated by dicyanomethylene. The present invention also provides a process to synthesize successfully pyrrolo-pyrroledione-thiophenequinones through zero-valent Pd catalyzed Takahashi coupling reaction and oxidizing condition of saturated bromine water. This process has a short procedure and a low cost. This kind of compounds has the following unique advantages because of the introduction of the pyrrolo-pyrroledione group: first, various substituents (for example, various type of alkyl chains: linear chains or branched chains, etc) can be introduced on the two nitrogen atoms of pyrrolo-pyrroledione unit so as to regulate the solubility of the compounds and improve the accumulation orderliness of molecules in thin film; second, the synthesis process is quite simple; and third, the quinone structure terminated by dicyanomethylene is maintained so that the compounds have lower LUMO energy level in order to meet the requirements for an air-stable n-type semiconductor. Hence, this kind of compounds is an excellent semiconductor material for n-type organic field effect transistors and has excellent field effect performances. The electronic mobility of said compounds is more than 0.1 cm²V⁻¹s⁻¹ (the electronic mobility of field effect device of semiconductor layer prepared through vacuum evaporation is up to 0.3 cm²V⁻¹s⁻¹; and the electronic mobility of field effect device of semiconductor layer prepared through spin coating is up to 0.35 cm²V⁻¹s⁻¹). The on/off current ratio of the compounds reaches up to 10⁵, and this kind of compounds has stable performance in air. Thus these compounds have important application values.

### Brief Description of the Drawings

Figure 1 is a synthetic scheme of pyrrolo-pyrroledione-thiophenequinone molecules.
Figure 2 is a schematic diagram for field effect transistor with pyrrolo-pyrroledione-thiophenequinone molecule as semiconductor active layer.
Figure 3 is the output characteristic curve of field effect transistor having the material of Example 1.
Figure 4 is the transfer characteristic curve of field effect transistor with the material of Example 1 under the source and drain voltage of 100 V.
Figure 5 is the output characteristic curve of field effect transistor having material of Example 2.
Figure 6 is the transfer characteristic curve of field effect transistor with the material of Example 2 under the source and drain voltage of 100 V.

### Embodiments of the Invention

The present invention will be further explained in combination with working Examples, but the present invention is not limited to these Examples. The methods mentioned all are conventional methods except stated otherwise. The materials mentioned all are commercially available except stated otherwise. The output characteristic curve and transfer characteristic curve under the source and drain voltages of 100 V of n-type organic field effect transistor prepared in the following Examples were tested by Keithley 4200 type semiconductor tester and 6150 type probe station under air.

### Example 1: Synthesis of Compound (a) and Preparation of n-type Organic Field Effect Transistor

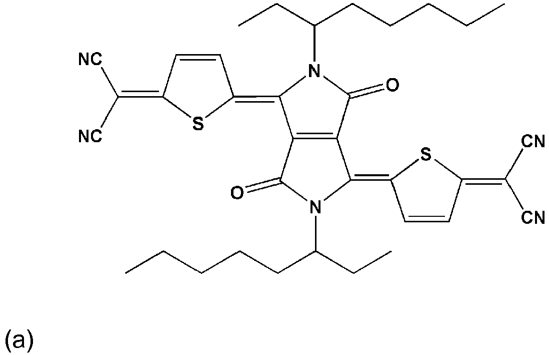

### 1) Synthesizing Compound (a):

Under the protection of nitrogen, sodium hydride (0.111g, 4.63 mmol) was suspended in ethylene glycol dimethyl ether (10 mL), and the temperature was decreased to 0°C. Malononitrile (0.092 g, 1.39 mmol) was added slowly into the system and then stirred for 10 minutes at this temperature. The icebath was removed and the system was warmed to room temperature and stirred for 30 minutes. α-bromine substituted pyrrolo-pyrroledione-thiophene oligomer precursor wherein R is 2-ethyl-n-hexyl alkyl chain (0.393 g, 0.58 mmol) and palladium(0) tetrakis(triphenylphosphine) (0.067g, 0.058 mmol) were added into the system sequentially, and the system was heated to reflux (the temperature was 100°C). With the passing of time, a dark purple precipitate separated out from the system one hour later, indicating the that divalent anionic intermediate was formed. The system was cooled to room temperature 4.5 hours later, and then cooled to 0°C by ice bath. Saturated bromine water (25 mL) was added to the system dropwise, and then the system was stirred for 30 minutes at this temperature. The system was warmed to room temperature (25°C) and stirred for 2.5 hours. Dichloromethane (50 mL × 3) was added to the system for extraction, and the organic phases were combined. The combined organic phase was washed with saturated sodium chloride aqueous solution, and dried with anhydrous magnesium sulfate. The organic phase was filtered under vacuum, rotary evaporated to dryness and the resulting residue was purified through column chromatography (petroleum ether: dichloromethane=1:2). After recrystallization with dichloromethane, 0.230 g olive green powered product was obtained, yield 61%, which was Compound (a) covered by general formula I provided in the present invention.

### Melting Point: 329 °C

The structural data of Compound (a) is shown as follows:
¹H NMR (400 MHz, CDCl₃) δ 9.42 (d, J = 5.8Hz, 2H), 7.38 (d, J = 5.8Hz, 2H), 3.99 (m, 4H), 1.90 (m, 2H), 1.42-1.27 (m, 16H), 0.97-0.88 (m, 12H); high resolution mass analysis (m/e): calculated value 650.2498, measured value 650.2504.

It can be seen that the compound is structurally correct and is the target compound.

### 2) Preparing n-type organic field effect transistor:

This product was evaporated onto a Si wafer substrate with a silica layer (thickness 500 nm) modified by n-octadecyltrichlorosilane (OTS) under the condition of vacuum degree of 4×10⁻⁴ Pa at the speed of 0.1-0.5 Å/s. The semiconductor layer was composed of Compound (a) covered by Formula I prepared in Example 1, with the thickness of 50 nm. Then gold electrodes as source electrode and drain electrode were deposited under vacuum, with thickness of 30 nm, to provide the n-type organic field effect transistor of the present invention.

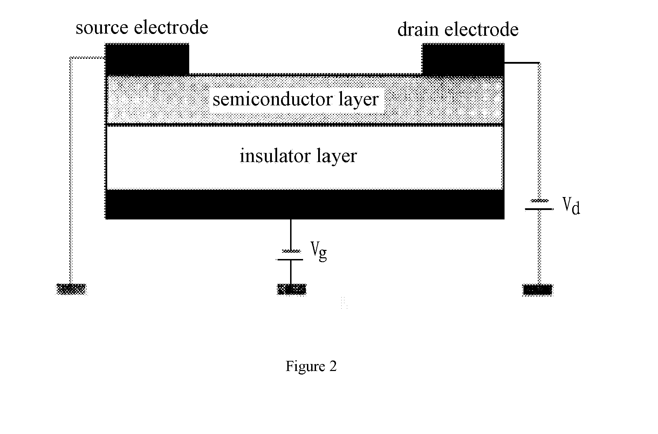

This n-type organic field effect transistor, as shown in Figure 2, was composed of, from bottom to up: substrate, insulator layer, semiconductor layer, and source electrode layer and drain electrode layer located in the same layer; said source electrode layer and drain electrode layer are not in contact with each other; wherein the material forming said semiconductor layer was Compound (a) covered by Formula I as prepared in Example 1.

The material forming the substrate was silicon wafer; the material forming said insulator layer was silica; the thickness of said insulator layer was 500 nm; the material forming said source electrode layer and drain electrode layer was gold; the thicknesses of both source electrode layer and drain electrode layer were 30 nm; and the thickness of said semiconductor layer was 50 nm.

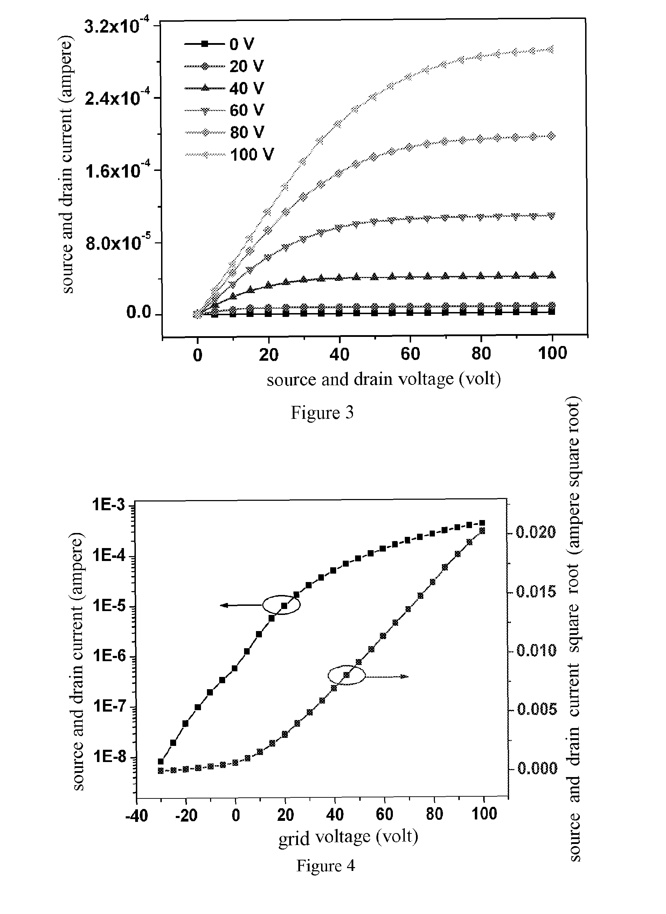

The organic field effect transistor prepared in this Example was tested by Keithley 4200 type semiconductor tester and 6150 type probe station under air. Figures 3 and 4 show the output characteristic curve and transfer characteristic curve under the source and drain voltage of 100 V of the field effect transistor prepared in said Example. It can be seen from the Figures that the field effect electronic mobility on the basis of this product is up to 0.3 cm²V⁻¹s⁻¹, and the on/off current ratio is up to 10⁵.

### Example 2: Synthesis of Compound (b) and preparation of n-type organic field effect transistor

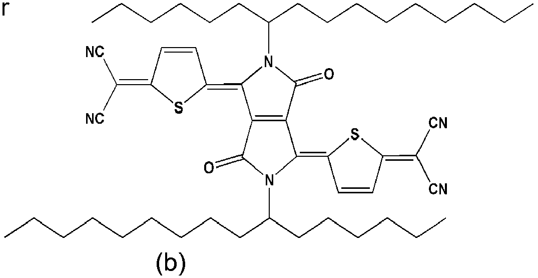

### 1) Synthesizing Compound (b):

Under the protection of nitrogen, sodium hydride (0.111g, 4.63 mmol) was suspended in ethylene glycol dimethyl ether (10 mL), and the temperature was decreased to 0°C. Malononitrile (0.092 g, 1.39 mmol) was added slowly into the system and then stirred for 10 minutes at this temperature. The icebath was removed and the system was warmed to room temperature and stirred for 30 minutes. α-bromine substituted pyrrolo-pyrroledione-thiophene oligomer precursor wherein R is 2-hexyl-n-decyl alkyl chain (0.522 g, 0.58 mmol) and palladium(0) tetrakis(triphenylphosphine) (0.067g, 0.058 mmol) were added into the system sequentially, and the system was heated to reflux (the temperature was 100°C). With the passing of time, a dark purple precipitate separated out from the system one hour later, indicating that divalent anionic intermediate was formed. The system was cooled to room temperature 4.5 hours later, and then cooled to 0°C by ice bath. Saturated bromine water (25 mL) was added to the system dropwise, and then the system was stirred for 30 minutes at this temperature. The system was warmed to room temperature (25°C) and stirred for 2.5 hours. Dichloromethane (50 mL × 3) was added to the system for extraction, and the organic phases were combined. The combined organic phase was washed with saturated sodium chloride aqueous solution, and dried with anhydrous magnesium sulfate. The organic phase was filtered under vacuum, rotary evaporated to dryness and the resulting residue was purified through column chromatography (petroleum ether:dichloromethane =1:1). After recrystallization with a mixture of dichloromethane and petroleum ether, 0.170 g olive green powered product was obtained, yield 34%, which was Compound (b) covered by general formula I provided in the present invention.

### Melting Point: 207 °C

The structural data of Compound (b) is shown as follows:
¹H NMR (400 MHz, CDCl₃) δ 9.42 (d, J = 5.6Hz, 2H), 7.38 (d, J = 5.7Hz, 2H), 3.99 (m, 4H), 1.92 (m, 2H), 1.37-1.25 (m, 48H), 0.97-0.88 (m, 12H); high resolution mass analysis (m/e): calculated value 874.5002, measured value 874.5012.

It can be seen that the compound is structurally correct and is the target compound.

### 2) Preparing n-type organic field effect transistor:

The product was formulated into a trichloromethane solution and was spin-coated uniformly onto a Si wafer substrate with a silica layer (thickness 500 nm) modified by n-octadecyltrichlorosilane (OTS) at the rate of 2000 rpm. The semiconductor layer was composed of Compound (b) covered by Formula I prepared in this Example, with the thickness of 50 nm. Then gold electrodes as source electrode and drain electrode were deposited under vacuum, with thickness of 30 nm, to provide the n-type organic field effect transistor of the present invention.

This n-type organic field effect transistor, as shown in Figure 2, was composed of, from bottom to up: substrate, insulator layer, semiconductor layer, and source electrode layer and drain electrode layer located in the same layer; said source electrode layer and drain electrode layer are not in contact with each other; wherein the material forming said semiconductor layer was Compound (b) covered by Formula I as prepared in Example 2.

The material forming the substrate was silicon wafer; the material forming said insulator layer was silica; the thickness of said insulator layer was 500 nm; the material forming said source electrode layer and drain electrode layer was gold; the thicknesses of both source electrode layer and drain electrode layer were 30 nm; and the thickness of said semiconductor layer was 50 nm.

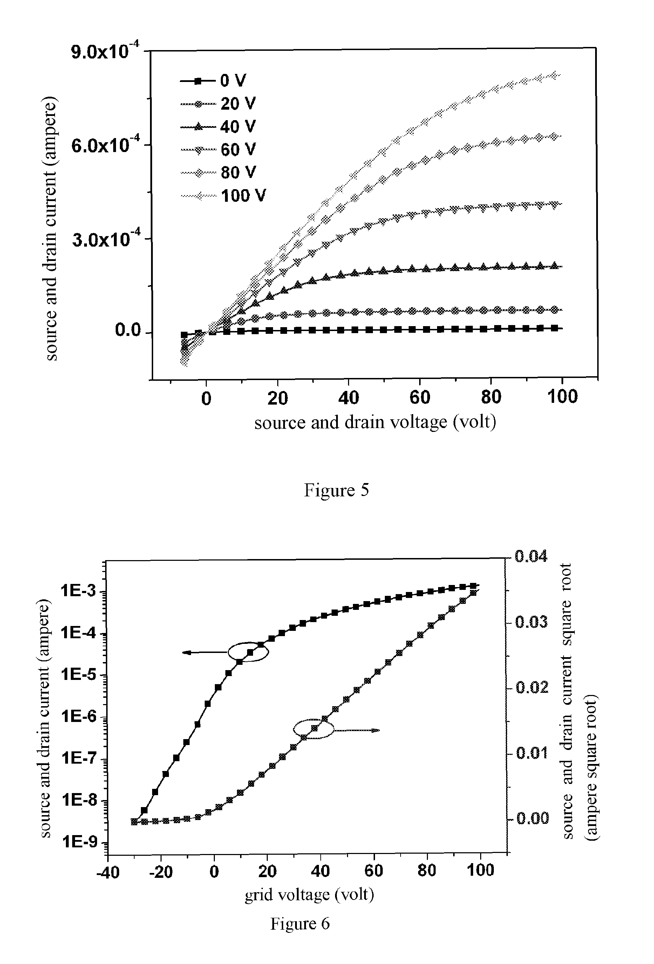

The organic field effect transistor prepared in this Example was tested by Keithley 4200 type semiconductor tester and 6150 type probe station under air. Figures 5 and 6 show the output characteristic curve and transfer characteristic curve under the source and drain voltages of 100 V of the field effect transistor prepared in said Example. It can be seen from the Figures that the field effect electronic mobility on the basis of this product is up to 0.35 cm²V⁻¹s⁻¹, and the on/off current ratio is up to 10⁵.

## Claims

1. A pyrrolo-pyrroledione-thiophenequinone compound represented by Formula I, 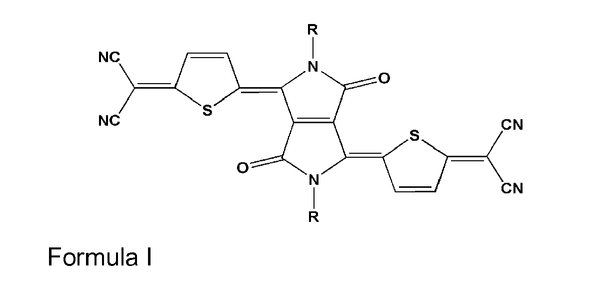 wherein, in Formula I, R is hydrogen, alkyl with a total number of 8 to 20 carbon atoms or alkoxy with a total number of 8 to 20 carbon atoms.

2. The compound according to Claim 1, **characterized in that** in Formula I, R is branched alkyl with a total number of 8 carbon atoms or branched alkyl with a total number of 16 carbon atoms.

3. A process for preparing the compound of Claim 1 or 2, comprising the steps of: under the catalysis of palladium(0) tetrakis(triphenylphosphine), mixing sodium hydride, α-bromine substituted pyrrolo-pyrroledione-thiophene oligomer represented by Formula II and malononitrile sodium salt uniformly to carry out nucleophilic substitution reaction to form a divalent anionic intermediate, and then adding saturated bromine water to the reaction system to carry out oxidation reaction, so as to provide the compound represented by Formula I; 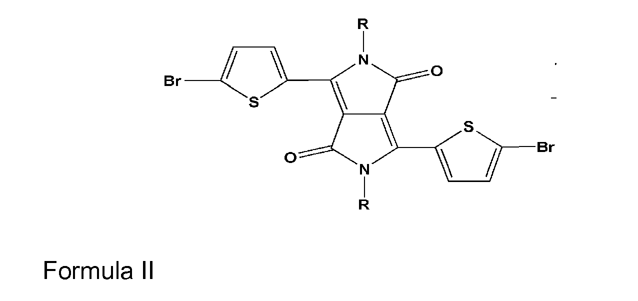 wherein, in Formula II, R is hydrogen, alkyl with a total number of 8 to 20 carbon atoms or alkoxy with a total number of 8 to 20 carbon atoms.

4. The process according to Claim 3, **characterized in that** in Formula II, R is branched alkyl with a total number of 8 carbon atoms or branched alkyl with a total number of 16 carbon atoms.

5. The process according to Claim 3 or 4, **characterized in that** the ratio of said sodium hydride, α-bromine substituted pyrrolo-pyrroledione-thiophene oligomer represented by Formula II, malononitrile sodium salt and saturated bromine water is 4.63-5.56 mmol: 0.58-0.61 mmol: 1.39-1.45 mmol: 25-30 mL, preferably 4.63 mmol: 0.58 mmol: 1.39 mmol: 25 mL.

6. The process according to any one of Claims 3-5, **characterized in that** in the step of nucleophilic substitution reaction, the temperature is 90-110°C, preferably 100°C, and the period is 4-6 hours, preferably 4.5 hours; in the step of oxidation reaction, the temperature is 0-25°C, preferably 25°C, and the period is 2-3 hours, preferably 2.5 hours.

7. The process according to any of Claims 3-6, **characterized in that** both the nucleophilic substitution reaction and the oxidation reaction are carried out in a solvent and under an inert atmosphere; wherein said solvent is selected from the group consisting of ethylene glycol dimethyl ether, tetrahydrofuran, N,N-dimethylformamide or a combination thereof dried by sodium, preferably ethylene glycol dimethyl ether; and said inert atmosphere is nitrogen atmosphere or argon atmosphere.

8. A semiconductor layer of an organic field effect transistor, wherein the semiconductor layer is formed from one or more compounds according to Claim 1 or 2.

9. A n-type organic field effect transistor which is composed of, from bottom to up: a substrate, an insulator layer, a semiconductor layer, and a source electrode layer and a drain electrode layer located in the same layer; said source electrode layer and drain electrode layer do not contact with each other; **characterized in that** the material forming said semiconductor layer is one or more compounds according to Claim 1 or 2.

10. The transistor according to Claim 9, **characterized in that** the material forming the substrate is selected from the group consisting of glass, ceramic, silicon wafer, or a combination thereof, preferably silicon wafer;
the material forming said insulator layer is selected from the group consisting of silica, n-octadecyltrichlorosilane modified silica, aluminium oxide, polyvinylpyrrolidone, polymethylmethacrylate, or a combination thereof, preferably silica; wherein the thickness of said insulator layer is 300-500 nm, preferably 500 nm; the material forming said source electrode layer and drain electrode layer is selected from the group consisting of gold, silver, aluminum, or a combination thereof, preferably gold; and wherein the thicknesses of source electrode layer or drain electrode layer is 20-30 nm, preferably 30 nm; and
the thickness of said semiconductor layer is 50-80 nm, preferably 50 nm.
